# FASCICULE DE BREVET EUROPEEN

(11) **EP 1 397 113 B1**
(45) Date de publication et mention de la délivrance du brevet: **11.10.2006**
(21) Numéro de dépôt: 02738296.9
(22) Date de dépôt: 31.05.2002
(51) Int. Cl.: A61K 8/25, A61K 8/41, A61Q 5/06

(54) **COMPOSITION EN AEROSOL COMPRENANT DES PARTICULES DE SILICATE ET DES TENSIOACTIFS**
SILIKATPARTIKEL UND TENSIDE ENTHALTENDE AEOROSOLZUSAMMENSETZUNG
AEROSOL COMPOSITION COMPRISING SILICATE PARTICLES AND SURFACTANTS

(30) Priorité: 31.05.2001 FR 0107154
(43) Date de publication de la demande: 17.03.2004
(73) Titulaire: L'ORÉAL, 75008 Paris (FR)
(72) Inventeur: BELLI, Emmanuelle, F-92600 Asnieres (FR); GILLES, Audrey, F-78140 Velizy (FR)
(74) Mandataire: Bourdeau, Françoise
(86) Numéro de dépôt international: PCT/FR2002/001849
(87) Numéro de publication internationale: WO 2002/096380

(56) Documents cités:
- WO-A-00/21495
- DATABASE CHEMICAL ABSTRACTS [en ligne] STN; access number: 127: 23 481, XP002193086 & JP 09 087145 A (TOYO AEROSOL IND. CO.) 31 mars 1997 (1997-03-31)
- DATABASE CHEMICAL ABSTRACTS [en ligne] STN; access number: 130: 342 765, XP002193087 & JP 11 100308 A (POLA CHEM. IND., INC.) 13 avril 1999 (1999-04-13)

## Description

La présente invention est relative à une composition conditionnée dans un dispositif aérosol comprenant un agent propulseur et une phase liquide qui contient, dans un milieu cosmétiquement acceptable, des particules de silicate, à un procédé de traitement cosmétique des cheveux et à une utilisation en tant que produit de coiffage.

Les produits coiffants sont habituellement utilisés pour construire, structurer la coiffure et lui apporter une tenue durable. Pour cela, des polymères filmogènes sont introduits dans un milieu cosmétiquement acceptable. Cependant, certains polymères entraînent un durcissement de la chevelure et/ou un caractère collant. De ce fait, les cheveux sont souvent collés entre eux, et le démêlage induit une destruction de la forme de la chevelure.

Pour remédier à ces inconvénients, il a déjà été proposé l'utilisation de poudre solide dans les compositions cosmétiques, telles que des poudres d'oxydes métalliques. Par exemple, le brevet US 3 819 827 décrit des produits pour la mise en plis des cheveux comprenant de 0,2 à 6 % en poids de particules d'oxyde d'aluminium présentant une taille de particule d'environ 30 mµ.

De la même manière, la demande de brevet EP 1 005 849 décrit l'utilisation de fines poudres solides inorganiques, comme les poudres de carbonate de calcium, d'hydroxyde d'aluminium, de carbonate de magnésium, de phosphate de calcium, d'oxalate de calcium ou de sulfate de baryum, dans des compositions cosmétiques capillaires contenant au moins deux phases liquides. Il est précisé que de telles compositions procurent sur les cheveux des effets spécialement avantageux lorsque la composition est conditionnée, sans gaz propulseur, dans un dispositif susceptible de pulvériser là composition sur les cheveux.

Le brevet DE 25 42 338 décrit des lotions et des sprays, sans propulseur, pour la fixation des cheveux comprenant des particules de silicates, dans un milieu alcoolique ou hydroalcoolique, la concentration en silicates étant comprise entre 0,1 et 4 %.

La Demanderesse a trouvé, de manière surprenante et inattendue, qu'en choisissant, comme particules solides, des particules de silicate, il est possible d'obtenir de bonnes propriétés cosmétiques et d'obtenir un coiffage sans coller ni surcharger les cheveux, dans la mesure où la composition est conditionnée dans un dispositif particulier, à savoir un dispositif aérosol, quand la composition cosmétique comprend, en outre au moins un tensioactif.

L'invention a pour objet une composition cosmétique, notamment capillaire, conditionnée dans un dispositif aérosol contenant un agent propulseur et une phase liquide qui comprend, dans un milieu cosmétiquement acceptable :
(i) des particules solides contenant au moins 10 % en poids d'au moins un silicate, et
(ii) au moins un tensioactif ;
lesdites particules solides contenant moins dé 1 % d'aluminium.

Un autre objet de la présente invention consiste en un procédé de traitement cosmétique des cheveux mettant en oeuvre la composition selon l'invention.

L'invention a encore pour objet l'utilisation de la composition en cosmétique capillaire, notamment pour fixer et/ou maintenir les cheveux dans une forme souhaitée.

L'invention vise encore l'utilisation, en cosmétique capillaire, notamment pour fixer et/ou maintenir les cheveux dans une forme souhaitée, d'une composition cosmétique conditionnée dans un dispositif aérosol contenant un agent propulseur et une phase liquide qui comprend, dans un milieu cosmétiquement acceptable, des particules solides contenant au moins 10 % en poids d'au moins un silicate, lesdites particules solides contenant moins de 1 % en poids d'aluminium.

De préférence, les particules solides contiennent entre 0 et 1 % d'aluminium, et plus préférentiellement entre 0 et 0,5 % d'aluminium.

D'autres objets, caractéristiques, aspects et avantages de l'invention apparaîtront encore plus clairement à la lecture de la description et des divers exemples qui suivent.

### PARTICULES

Avantageusement, les particules solides présentent une taille primaire moyenne en nombre comprise entre 2 nm et 1 µm.

Les particules contenant au moins 10 % en poids d'au moins un silicate présentent, de préférence, une taille primaire moyenne en nombre comprise entre 5 et 500 nm, et plus préférentiellement entre 10 et 250 nm.

Les particules selon l'invention peuvent avoir une forme quelconque, par exemple la forme de sphères, de paillettes, d'aiguilles, de plaquettes ou des formes totalement aléatoires.

De préférence, il s'agit de silicates de sodium, de magnésium et/ou de lithium, comme les composés commercialisés par la Société Laporte sous les dénominations LAPONITE XLG et LAPONITE XLS.

Au sens de la présente invention, on entend par *"taille primaire de particule",* la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Dans le cas où les particules sont formées par du silicate et d'autres charges, le silicate se trouve à l'état libre et ne forme pas de liaisons chimiques avec les autres charges. Il s'agit alors d'un alliage entre le silicate et d'autres charges, notamment avec des oxydes de métaux ou de métalloïdes, en particulier obtenu par fusion thermique de ces différents constituants.

Lorsque les particules contenant au moins 10 % en poids d'au moins un silicate comprennent, en outre, un oxyde de métal ou de métalloïde, celui-ci est notamment choisi parmi l'oxyde de silicium, de bore, d'aluminium.

De préférence, les particules contiennent au moins 50 % en poids de silicate, mieux encore au moins 70 % en poids, et les particules constituées à plus de 90 % en poids de silicate sont particulièrement préférées selon la présente invention.

Le silicate convenant dans les compositions de la présente invention peut être d'origine naturelle ou peut être d'origine synthétique.

Les particules contenant du silicate selon l'invention sont, notamment, utilisées en une quantité comprise entre 0,01 % et 10 % en poids, et de préférence entre 0,5 % et 3 % en poids par rapport au poids total de la composition.

La composition selon l'invention peut également contenir d'autres types de particules, par exemple, des particules d'oxyde de titane ou de zinc, d'aluminium.

### TENSIOACTIF

Les tensioactifs convenant à la mise en oeuvre de la présente invention sont notamment les suivants :

### (i) Tensioactif(s) anionique(s):

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels d'alcalinoterreux (de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant dé 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s):

Les agents tensioactifs non ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL®, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R_{2'}-CONHCH₂CH₂-N(B)(C) (3)

dans laquelle :
B représente -CH₂CH₂OX', C représente -(CH₂)_{z}-Y', avec z = 1 où 2,
X' désigné le groupement -CH₂CH₂-COOH ou un atome d'hydrogène
Y' désigne -COOH ou le radical -CH₂- CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique ou non ionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂₋C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA sous la dénomination commerciale "MIRANOL® C2M CONC" en solution aqueuse à 38 % de matière active ou sous la dénomination MIRANOL® C32;
- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidobétaïnes telles que la TEGOBETAINE® F50 commercialisée par la société GLODSCHMIDT.

### TENSIOACTIFS CATIONIQUES

La composition selon l'invention comprend un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux qui présentent la formule générale (VI) suivante : 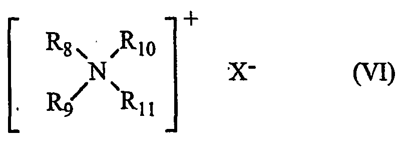
dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (VII) suivante : 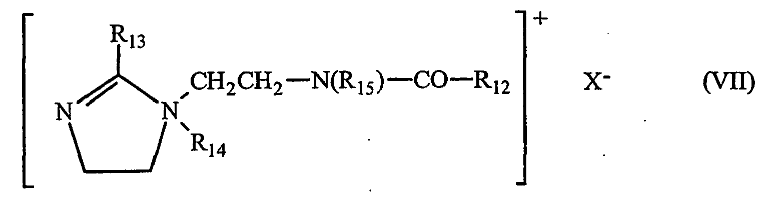
dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT® W 75 par la société REWO ;
- les sels de diammonium quaternaire de formule (VIII) : 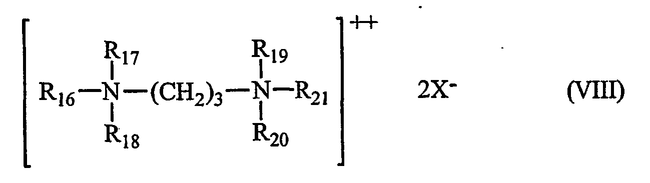
dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IX) suivante : 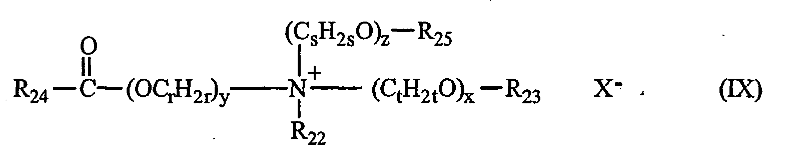
dans laquelle :
R₂₂ est choisi parmi les radicaux alkyles en C₁-C₆ et les radicaux hydroxyalkyles ou dihydroxyalkyles en C₁-C₆ ;
R₂₃ est choisi parmi :
   - le radical 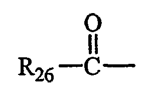
   - les radicaux R₂₇ hydrocarbonés en C₁-C₂₂, linéaires ou ramifiés, saturés ou insaturés,
   - l'atome d'hydrogène,
R₂₅ est choisi parmi :
   - le radical 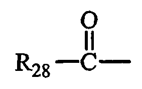
   - les radicaux R₂₉ hydrocarbonés en C₁-C₆, linéaires ou ramifiés, saturés ou insaturés,
   - l'atome d'hydrogène,
R₂₄, R₂₆ et R₂₈, identiques ou différents, sont choisis parmi les radicaux hydrocarbonés en C₇-C₂₁, linéaires ou ramifiés, saturés ou insaturés ;
r, s et t, identiques ou différents, sont des entiers valant de 2 à 6 ;
y est un entier valant de 1 à 10 ;
x et z, identiques ou différents, sont des entiers valant de 0 à 10 ;
X⁻ est un anion simple ou complexe, organique ou inorganique ;
sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, là somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IX) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 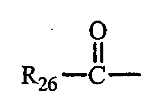
   - les radicaux méthyle, éthyle ou hydrocarbonés en C₁₄-C₂₂,
   - l'atome d'hydrogène ;
- R₂₅ est choisi parmi :
   - le radical 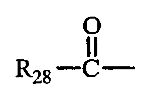
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IX) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention contient de préférence un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthyl-ammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

Les tensioactifs cationiques particulièrement préférés dans la composition de l'invention sont choisis parmi les sels d'ammonium quaternaire, et en particulier parmi le chlorure de béhényltriméthylammonium et le chlorure de palmitylamidopropyltriméthylammonium.

La composition selon l'invention comprend de préférence le ou les tensioactifs en une quantité comprise entre 0,1 et 10 % en poids, mieux encore entre 0,5 et 8 % en poids, et encore plus préférentiellement entre 1 et 5 % en poids par rapport au poids total de la composition.

Avantageusement, on choisit, comme tensioactif, un tensioactif amphotère ou non ionique.

### COMPOSITION

Le milieu cosmétiquement acceptable comprend de l'eau et/ou au moins un solvant cosmétiquement acceptable notamment choisi parmi les alcools inférieurs en C₁-C₄, comme l'éthanol, l'isopropanol, le tertio-butanol et le n-butanol ; les polyols, comme le propylèneglycol ; les éthers de polyols; l'acétone et leurs mélanges. Le solvant particulièrement préféré dans l'invention est l'éthanol.

La quantité d'eau est, de préférence, comprise entre 10 et 95 %, et encore plus préférentiellement entre 15 et 45 %.

De préférence, l'agent propulseur est choisi parmi le diméthyléther, les alcanes en C₃ à C₅, comme le n-butane, le propane, l'isobutane, les hydrocarbures halogénés, comme le le 1,1-difluoroéthane, les mélanges de diméthyléther et d'alcanes en C₃₋₅, les mélanges de 1,1-difluoroéthane et de diméthyléther et/ou d'alcanes en C₃₋₅.

Plus préférentiellement, l'agent propulseur est choisi parmi le diméthyléther, les alcanes en C₃ à C₅ et leurs mélanges.

La composition selon l'invention peut comprendre des additifs cosmétiques usuels choisis notamment parmi les agents adhésifs, les agents réducteurs comme les thiols, les corps gras, les agents épaississants, les adoucissants, les agents anti-mousse, les filtres, les agents antiperspirants, les agents acidifiants, les agents alcalinisants, les colorants, les pigments, les parfums, les conservateurs, les polymères anioniques, cationiques, non ioniques ou amphotères fixants ou non fixants, les silicones volatiles ou non, modifiées ou non, solubles ou non solubles, les huiles végétales, animales, minérales ou de synthèse, les parfums, les protéines et les vitamines, le glycérol et leurs mélanges.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.

L'agent propulseur est notamment présent en une quantité comprise entre 2 et 90 % en poids, de préférence entre 4 et 80 %, mieux encore entre 30 et 70 % en poids par rapport au poids total de la composition.

Les compositions conformes à l'invention sont conditionnées dans un dispositif aérosol usuel en cosmétique. Les compositions pulvérisées peuvent se présenter sous la forme d'un spray ou d'une mousse.

Les compositions conformes à l'invention, pulvérisées à partir du dispositif aérosol, peuvent être utilisées en application rincée ou non, comme compositions de fixation et/ou de maintien des cheveux, compositions de soin de cheveux, shampooings, compositions de conditionnement des cheveux, telles que des compositions destinées à apporter de la douceur aux cheveux ou encore des compositions de maquillage des cheveux.

La présente invention concerne également un procédé de traitement cosmétique des cheveux qui consiste à appliquer une quantité efficace d'une composition telle que décrite ci-dessus, sur les cheveux et à rincer ou non après un éventuel temps de pose.

Selon un mode de réalisation préféré de l'invention, la composition, pulvérisée à partir du dispositif aérosol, peut être utilisée comme produit de coiffage non rincé.

En particulier, les compositions conformes à l'invention sont utilisées pour la mise en forme et/ou le maintien de la chevelure.

Les exemples suivants illustrent la présente invention et ne doivent être considérés en aucune manière comme limitant l'invention.

### Exemples

On a préparé trois produits de coiffage à partir des ingrédients suivants, les pourcentages étant exprimés en poids :

### Exemple 1 :

| | |
|---|---|
| Laponite XLG (Laporte) | 0.8% |
| Tégo-bétaïne HS (Goldschmidt) | 0.4% |
| Brij 30 (Uniqema) | 0.2% |
| Viscophobe DB 1000 (Amerchol) | 0.86% |
| Conservateurs | qs |
| Aérogaz 3.2 (1) | 6% |
| Eau | qsp 100% |

| | |
|---|---|
| (1) mélange d'isobutane, propane, butane commercialisé par Atochem | |

### Exemple 2 :

| | |
|---|---|
| Laponite XLG (Laporte) | 0.5% |
| Tégo-bétaïne HS (Goldschmidt) | 0.5% |
| Brij 30 (Uniqema) | 0.2% |
| Conservateurs | qs |
| Butane 3.2 | 6% |
| Eau | qsp 100% |

### Exemple 3 :

| | |
|---|---|
| Laponite XLG (Laporte) | 1 % |
| Tégo-bétaïne HS (Goldschmidt) | 0.5% |
| Brij 30 (Uniqema) | 0.2% |
| Conservateurs | qs |
| Butane 3.2 | 6% |
| Eau | qsp 100% |

Ces produits se présentent sous forme de mousse aérosol et sont appliqués, de préférence, sur cheveux humides. Après mise en forme, on observe un bon effet coiffant et un maintien durable sans inconvénient cosmétique.

## Revendications

1. Composition cosmétique, notamment capillaire, conditionnée dans un dispositif aérosol contenant un agent propulseur et une phase liquide qui comprend, dans un milieu cosmétiquement acceptable :
(i) des particules solides contenant au moins 10 % en poids d'au moins un silicate, et
(ii) au moins un tensioactif ;
lesdites particules solides contenant moins de 1 % en poids d'aluminium.

2. Composition selon la revendication 1, **caractérisée par le fait que** le silicate est choisi parmi les silicates de sodium, de magnésium et/ou de lithium.

3. Composition selon l'une quelconque des revendications précédentes, **caractérisée par le fait que** les particules solides présentent une taille primaire moyenne en nombre comprise entre 2 nm et 1 µm.

4. Composition selon l'une quelconque des revendications précédentes, **caractérisée en ce que** les particules contenant au moins 10 % en poids d'au moins un silicate présentent une taille primaire moyenne en nombre comprise entre 5 et 500 nm, et plus préférentiellement entre 10 et 250 nm.

5. Composition selon l'une quelconque des revendications précédentes, **caractérisée en ce que** les particules contiennent au moins 50 % en poids de silicate, de préférence 70 % en poids, et plus préférentiellement encore, plus de 90 % en poids de silicate.

6. Composition selon l'une quelconque des revendications précédentes, **caractérisée en ce que** les particules contenant du silicate sont présentes en une quantité comprise entre 0,01 % et 10 % en poids, et de préférence entre 0,5 % et 3 % en poids par rapport au poids total de la composition.

7. Composition selon l'une quelconque des revendications précédentes, **caractérisée par le fait qu'**elle comprend de préférence le ou les tensioactifs en une quantité comprise entre 0,1 et 10 % en poids, mieux encore entre 0,5 et 8 % en poids, et encore plus préférentiellement entre 1 et 5 % en poids par rapport au poids total de la composition.

8. Composition selon l'une quelconque des revendications précédentes, **caractérisée par le fait que** le tensioactif est tensioactif amphotère ou non ionique.

9. Composition selon l'une quelconque des revendications précédentes, **caractérisée en ce que** l'agent propulseur est choisi parmi le diméthyléther, les alcanes en C₃ à C₅, comme le n-butane, le propane, l'isobutane, les hydrocarbures halogénés, comme le le 1,1-difluoroéthane, les mélanges de diméthyléther et d'alcanes en C₃₋₅, les mélanges de 1,1-difluoroéthane et de diméthyléther et/ou d'alcanes en C₃₋₅.

10. Composition selon la revendication précédente, **caractérisée en ce que** l'agent propulseur est choisi parmi le diméthyléther et les alcanes en C₃ à C₅ et leurs mélanges.

11. Composition selon l'une quelconque des revendications précédentes, **caractérisée en ce que** l'agent propulseur est présent en une quantité comprise entre 2 et 90 % en poids, de préférence entre 4 et 80 %, mieux encore entre 30 et 70 % en poids par rapport au poids total de la composition.

12. Composition selon l'une quelconque des revendications précédentes, **caractérisée en ce qu'**elle comprend des additifs cosmétiques usuels choisis parmi les agents adhésifs, les agents réducteurs comme les thiols, les corps gras, les agents épaississants, les adoucissants, les agents anti-mousse, les filtres, les agents antiperspirants, les agents acidifiants, les agents alcalinisants, les colorants, les pigments, les parfums, les conservateurs, les polymères anioniques, cationiques, non ioniques ou amphotères fixants ou non fixants, les silicones volatiles ou non, modifiées ou non, solubles ou non solubles, les huiles végétales, animales, minérales ou de synthèse, les parfums, les protéines et les vitamines, le glycérol et leurs mélanges.

13. Procédé de traitement cosmétique des cheveux mettant en oeuvre une composition selon l'une quelconque des revendications 1 à 12.

14. Utilisation d'une composition selon l'une quelconque des revendications 1 à 12 en cosmétique capillaire, notamment pour la mise en forme et/ou le maintien de la chevelure.

## Claims

1. Cosmetic composition, especially a hair composition, packaged in an aerosol device containing a propellant and a liquid phase, which comprises, in a cosmetically acceptable medium:
(i) solid particles containing at least 10% by weight of at least one silicate, and
(ii) at least one surfactant;
said solid particles containing less than 1% by weight of aluminium.

2. Composition according to Claim 1, **characterized in that** the silicate is chosen from sodium, magnesium and/or lithium silicates.

3. Composition according to either of the preceding claims, **characterized in that** the solid particles have a number-average primary size of between 2 nm and 1 µm.

4. Composition according to any one of the preceding claims, **characterized in that** the particles containing at least 10% by weight of at least one silicate have a number-average primary size of between 5 and 500 nm and more preferably between 10 and 250 nm.

5. Composition according to any one of the preceding claims, **characterized in that** the particles contain at least 50% by weight of silicate, preferably 70% by weight and even more preferably more than 90% by weight of silicate.

6. Composition according to any one of the preceding claims, **characterized in that** the silicate-containing particles are present in an amount of between 0.01% and 10% by weight and preferably between 0.5% and 3% by weight relative to the total weight of the composition.

7. Composition according to any one of the preceding claims, **characterized in that** it preferably comprises the surfactant(s) in an amount of between 0.1% and 10% by weight, better still between 0.5% and 8% by weight and even more preferably between 1% and 5% by weight relative to the total weight of the composition.

8. Composition according to any one of the preceding claims, **characterized in that** the surfactant is an amphoteric or nonionic surfactant.

9. Composition according to any one of the preceding claims, **characterized in that** the propellant is chosen from dimethyl ether, C₃ to C₅ alkanes, for instance n-butane, propane or isobutane, halohydrocarbons, for instance 1,1-difluoroethane, mixtures of dimethyl ether and of C₃₋₅ alkanes, and mixtures of 1,1-difluoroethane and of dimethyl ether and/or of C₃₋₅ alkanes.

10. Composition according to the preceding claim, **characterized in that** the propellant is chosen from dimethyl ether and C₃ to C₅ alkanes, and mixtures thereof.

11. Composition according to any one of the preceding claims, **characterized in that** the propellant is present in an amount of between 2% and 90% by weight, preferably between 4% and 80% by weight and better still between 30% and 70% by weight relative to the total weight of the composition.

12. Composition according to any one of the preceding claims, **characterized in that** it comprises common cosmetic additives chosen from adhesives, reducing agents, for instance thiols, fatty substances, thickeners, softeners, antifoams, screening agents, antiperspirants, acidifying agents, basifying agents, dyes, pigments, fragrances, preserving agents, anionic, cationic, nonionic or amphoteric, fixing or nonfixing polymers, volatile or nonvolatile, modified or unmodified, soluble or insoluble silicones, plant, animal, mineral or synthetic oils, fragrances, proteins, vitamins and glycerol, and mixtures thereof.

13. Cosmetic hair treatment process using a composition according to any one of Claims 1 to 12.

14. Use of a composition according to any one of Claims 1 to 12 in hair cosmetics, especially for shaping and/or holding the hair.

## Patentansprüche

1. Kosmetische Zusammensetzung, insbesondere für die Haarbehandlung, die in einem Aerosolbehälter konfektioniert ist, der ein Treibmittel und eine flüssige Phase enthält, welche in einem kosmetisch akzeptablen Medium aufweist:
(i) feste Partikel, die mindestens 10 Gew.-% mindestens eines Silicats enthalten, und
(ii) mindestens einen grenzflächenaktiven Stoff;
wobei die festen Partikel weniger als 1 % Aluminium enthalten.

2. Zusammensetzung nach Anspruch 1, **dadurch gekennzeichnet, dass** das Silicat unter den Natriumsilicaten, Magnesiumsilicaten und/oder Lithiumsilicaten ausgewählt ist.

3. Zusammensetzung nach einem der vorhergehenden Ansprüche, **dadurch gekennzeichnet, dass** die festen Partikel eine zahlenmittlere Primärgröße von 2 nm bis 1 µm aufweisen.

4. Zusammensetzung nach einem der vorhergehenden Ansprüche, **dadurch gekennzeichnet, dass** die Partikel, die mindestens 10 Gew.-% mindestens eines Silicats enthalten, eine zahlenmittlere Primärgröße von 5 bis 500 nm und vorzugsweise 10 bis 250 nm aufweisen.

5. Zusammensetzung nach einem der vorhergehenden Ansprüche, **dadurch gekennzeichnet, dass** die Partikel mindestens 50 Gew.-% Silicat, vorzugsweise 70 Gew.-% Silicat und noch bevorzugter mehr als 90 Gew.-% Silicat enthalten.

6. Zusammensetzung nach einem der vorhergehenden Ansprüche, **dadurch gekennzeichnet, dass** die Partikel, die Silicat enthalten, in einer Menge von 0,01 bis 10 Gew.-% und vorzugsweise 0,5 bis 3 Gew.-%, bezogen auf das Gesamtgewicht der Zusammensetzung, enthalten sind.

7. Zusammensetzung nach einem der vorhergehenden Ansprüche, **dadurch gekennzeichnet, dass** sie vorzugsweise den grenzflächenaktiven Stoff oder die grenzflächenaktiven Stoffe in einer Menge von 0,1 bis 10 Gew.-%, besser 0,5 bis 8 Gew.-% und noch bevorzugter 1 bis 5 Gew.-%, bezogen auf das Gesamtgewicht der Zusammensetzung, enthält.

8. Zusammensetzung nach einem der vorhergehenden Ansprüche, **dadurch gekennzeichnet, dass** der grenzflächenaktive Stoff ein amphoterer oder nichtionischer grenzflächenaktiver Stoff ist.

9. Zusammensetzung nach einem der vorhergehenden Ansprüche, **dadurch gekennzeichnet, dass** das Treibmittel unter Dimethylether, C₃₋₅-Alkanen, wie *n*-Butan, Propan, Isobutan, halogenierten Kohlenwasserstoffen, wie 1,1-Difluorethan, Gemischen aus Dimethylether und C₃₋₅-Alkanen, Gemischen aus 1,1-Difluorethan und Dimethylether und/oder C₃₋₅-Alkanen ausgewählt ist.

10. Zusammensetzung nach dem vorhergehenden Anspruch, **dadurch gekennzeichnet, dass** das Treibmittel unter Dimethylether und C₃₋₅-Alkanen und deren Gemischen ausgewählt ist.

11. Zusammensetzung nach einem der vorhergehenden Ansprüche, **dadurch gekennzeichnet, dass** das Treibmittel in einer Menge von 2 bis 90 Gew.-%, vorzugsweise 4 bis 80 Gew.-% und besser 30 bis 70 Gew.-%, bezogen auf das Gesamtgewicht der Zusammensetzung, enthalten ist.

12. Zusammensetzung nach einem der vorhergehenden Ansprüche, **dadurch gekennzeichnet, dass** sie übliche kosmetische Zusatzstoffe enthält, die unter den Haftmitteln, Reduktionsmitteln, wie Thiolen, Fettsubstanzen, Verdickungsmitteln, beruhigenden Stoffen, Schaumverhütungsmitteln, Filtern, Antiperspirantien, Ansäuerungsmitteln, Alkanisierungsmitteln, Farbmitteln, Pigmenten, Parfums, Konservierungsmitteln, anionischen, kationischen, nichtionischen oder amphoteren fixierenden oder nicht fixierenden Polymeren, flüchtigen oder nicht flüchtigen, modifizierten oder nicht modifizierten, löslichen oder nicht löslichen Siliconen, pflanzlichen, tierischen, mineralischen oder synthetischen Ölen, Parfums, Proteinen und Vitaminen, Glycerin und deren Gemischen ausgewählt sind.

13. Verfahren zur kosmetischen Behandlung der Haare unter Verwendung einer Zusammensetzung nach einem der Ansprüche 1 bis 12.

14. Verwendung einer Zusammensetzung nach einem der Ansprüche 1 bis 12 in der Haarkosmetik, insbesondere für die Formgebung und/oder Festigung der Frisur.
